# DEMANDE DE BREVET EUROPEEN

(11) **EP 1 559 398 A1**
(43) Date de publication de la demande: **03.08.2005**
(21) Numéro de dépôt: 05290175.8
(22) Date de dépôt: 27.01.2005
(51) Int. Cl.: A61K 7/06, A61K 7/48

(54) **Procédé de préparation d'une composition pour le traitement cosmétique des matiéres kératiniques à partir de fluide sous pression et de vitamines**

(30) Priorité: 29.01.2004 FR 0400850
(71) Demandeur: L'OREAL, 75008 Paris (FR)
(72) Inventeur: De la Mettrie, Roland, 78110 Le Vesinet (FR)
(74) Mandataire: Casalonga, Axel

(57) **Abrégé**

L'invention concerne un procédé de préparation d'une composition pour le traitement cosmétique des matières kératiniques, caractérisé en ce qu'il comprend une étape de percolation d'un fluide comprenant de la vapeur d'eau sous une pression d'au moins 3 bars au travers d'au moins une vitamine sous forme solide ou pâteuse.

## Description

La présente invention a pour objet un procédé de préparation d'une composition destinée au traitement cosmétique des matières kératiniques, en particulier de la peau et des fibres kératiniques humaines telles que les cheveux, ainsi qu'un procédé de traitement cosmétique des matières kératiniques à partir de cette composition.

En cosmétique, on cherche toujours à améliorer les propriétés cosmétiques des matières kératiniques. Il est ainsi connu d'utiliser dans des compositions pour application topique des actifs, et notamment des vitamines, permettant de lutter contre les dommages de la peau, et notamment les signes du vieillissement de la peau, de nourrir la peau et d'apporter à la peau et aux autres matières kératiniques traitées par ces compositions, tous les bienfaits liés à ces vitamines.

On sait qu'au cours du processus de vieillissement, il apparaît différents signes sur la peau humaine, très caractéristiques de ce vieillissement, se traduisant notamment par une modification de la structure et de la fonction cutanée. Les principaux signes cliniques de vieillissement cutané sont notamment les suivants : apparition de ridules puis de rides profondes en augmentation avec l'âge, et désorganisation du « grain » de la peau, c'est-à-dire que le micro-relief est moins régulier et présente un caractère anisotrope.

Par ailleurs, le teint de la peau est généralement modifié ; il apparaît plus pâle et plus jaune, ce qui semble être dû essentiellement à une désorganisation de la microcirculation (moins d'hémoglobine au niveau du derme papillaire). De nombreuses taches colorées apparaissent en surface, ce qui est dû à une mélanogénèse altérée. Un autre signe clinique de vieillissement est l'aspect sec et rêche de la peau qui est dû essentiellement à une desquamation plus importante, ces squames en diffractant les rayons lumineux participant aussi à l'aspect un peu gris du teint.

Ainsi, il est connu de traiter ces signes du vieillissement en utilisant des compositions cosmétiques ou dermatologiques contenant des vitamines.

Il est également connu de traiter des fibres kératiniques par des vitamines, lorsque les fibres ont été soumises à un traitement de réduction, comme une permanente, et/ou d'oxydation, comme une coloration, ou qu'elles ont été soumises à un stimulus extérieur, comme la lumière ou le chlore. Grâce à ce traitement, on obtient un ravivement de la couleur des fibres, de leur brillance, et une amélioration du lissage des fibres.

Il est toutefois nécessaire de disposer de compositions de traitement cosmétique des matières kératiniques dans lesquelles la solubilisation des vitamines est améliorée, et d'augmenter l'efficacité cosmétique des vitamines. De plus, les vitamines sont des composés instables, notamment en présence de lumière, d'un milieu oxydant, comme l'air, et/ou d'eau.

La Demanderesse a découvert de manière surprenante qu'en utilisant un nouveau procédé de préparation d'une composition de traitement cosmétique des matières kératiniques, on pouvait obtenir en un temps très court, inférieur à 2 minutes, des compositions plus ou moins concentrées en vitamines selon le besoin, notamment sans conservateur, permettant de surmonter les problèmes de solubilité, de stabilité et d'efficacité exposés ci-dessus.

Ce procédé est mis en oeuvre simplement. On fait passer un fluide sous pression, dont la température est de préférence supérieure ou égale à 30°C, de préférence allant de 30°C à 150°C, et encore plus préférentiellement allant de 40°C à 120°C pendant un laps de temps très court, inférieur à une minute, à travers au moins une vitamine sous forme solide ou pâteuse, de préférence sous forme solide, et encore de préférence sous forme pulvérulente.

Il permet d'utiliser sous forme anhydre des vitamines instables dans des compositions aqueuses, soit parce qu'elles réagissent avec l'eau, soit parce qu'elles réagissent en solution aqueuse avec des composés qui ne réagissent pas avec elles dans une composition anhydre.

Les compositions préparées selon ce procédé peuvent présenter une stabilité au stockage limitée, ce qui n'est pas ici un inconvénient puisque le procédé conduit à une composition prête à l'emploi, destinée à être utilisée rapidement après sa préparation, par exemple dans les 5 minutes suivant sa préparation, notamment après refroidissement jusqu'à une température cosmétiquement acceptable, de préférence inférieure à 60°C. La composition peut être utilisée jusqu'à une semaine après sa préparation, selon la vitesse de dégradation de la vitamine.

Etant donné le temps de préparation très court, les compositions de traitement cosmétique peuvent être préparées "à la demande" en mélangeant les composés actifs selon les propriétés cosmétiques recherchées.

Selon un autre mode de réalisation, les vitamines peuvent être conditionnées dans un dispositif prêt-à-l'emploi, et il n'est pas nécessaire de déterminer au préalable les concentrations des vitamines en solution, ce qui limite les erreurs de mesure de l'utilisateur.

En outre, le procédé selon l'invention permet d'éviter l'utilisation de flacons multi-compartiments, ce qui rend le procédé particulièrement économique et plus sûr pour l'utilisateur.

La composition ainsi obtenue peut être utilisée seule ou en mélange avec une autre composition.

Un avantage important de ce procédé de préparation est l'obtention de compositions conférant de bonnes propriétés cosmétiques. En particulier, les matières kératiniques traitées avec une composition obtenue par le procédé selon l'invention, sont protégées, nourries, renforcées, et présentent un aspect visuel particulièrement agréable.

L'invention a donc pour objet un procédé de préparation d'une composition de traitement cosmétique des matières kératiniques comprenant une étape de percolation d'un fluide sous une pression d'au moins 3 bars au travers d'au moins une vitamine sous forme solide ou pâteuse.

Un autre objet de l'invention est une composition susceptible d'être obtenue par le procédé selon l'invention.

L'invention a également pour objet l'utilisation notamment cosmétique de la composition obtenue selon le procédé de l'invention comme agent pour le traitement cosmétique des matières kératiniques.

L'invention a enfin pour objet un dispositif de conditionnement permettant de mettre en oeuvre le procédé de préparation de la présente invention.

D'autres objets, caractéristiques, aspects et avantages de l'invention apparaîtront encore plus clairement à la lecture de la description et des exemples qui suivent.

Selon l'invention, le procédé de préparation d'une composition de traitement cosmétique des matières kératiniques comprend une étape de percolation d'un fluide de préférence à une température au moins égale à 30°C, mieux encore allant de 30°C à 150°C, mieux encore de 40°C à 120°C, sous une pression d'au moins 3 bars (3.10⁵ Pa) au travers d'au moins une vitamine sous forme solide ou pâteuse.

La percolation est un mouvement de fluide à travers un milieu poreux saturé permettant le passage du fluide, sous l'action ou l'effet de la pression.

Le fluide peut être constitué par de la vapeur d'eau éventuellement accompagnée d'eau liquide, ou par un ou plusieurs solvants liquides et/ou gazeux cosmétiquement acceptables, notamment organiques, ou encore par un mélange de vapeur d'eau éventuellement accompagnée d'eau liquide, et d'un ou plusieurs solvants liquides et/ou gazeux cosmétiquement acceptables. De préférence, le fluide comprend au moins de la vapeur d'eau pouvant être accompagnée d'eau liquide, et encore plus préférentiellement il est de la vapeur d'eau pouvant être accompagnée d'eau liquide.

A titre de solvant organique, on peut par exemple citer les alcools inférieurs en C1-C4, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Par « composé insoluble dans l'eau », on entend tout composé qui, à une concentration supérieure ou égale à 0,1% en poids dans l'eau à 25°C, ne forme pas à l'oeil nu une solution isotrope transparente.

La vitamine est sous forme solide ou sous forme pâteuse, de préférence sous forme solide, et encore plus préférentiellement sous forme pulvérulente.

Par « forme pâteuse » au sens de la présente invention, on entend une consistance intermédiaire entre une phase solide et une phase liquide. La viscosité de cette phase pâteuse est de préférence supérieure à 0,1 Pa.s, et encore plus préférentiellement supérieure à 1 Pa.s, ceci à 25°C avec un taux de cisaillement de 10 s⁻¹.

Par vitamine au sens de l'invention, on entend les vitamines, leurs dérivés tels que les esters dont les glycérides, les glucosides, les sels et les éthers ; et leurs précurseurs.

Par matières kératiniques, on entend la peau, le cuir chevelu, les lèvres, et/ou les phanères tels que les ongles, les cils, les sourcils et les cheveux.

Le procédé de la présente invention peut être mis en oeuvre à partir d'un dispositif classique permettant de générer un fluide sous pression, à une température de préférence supérieure ou égale à 30°C, et encore plus préférentiellement allant de 30°C à 150°C. Un tel dispositif comprend une chambre résistant à la pression, équipée d'un bloc thermique, ainsi qu'un circuit d'acheminement du fluide produit vers la vitamine.

Selon un autre mode de réalisation, le dispositif comprend un réservoir de liquide(s) ainsi qu'une pompe permettant l'acheminement du ou des liquides vers la chambre.

Le liquide contenu dans le réservoir est soit de l'eau, soit un solvant cosmétiquement acceptable ou un mélange de plusieurs solvants cosmétiquement acceptables, ou encore un mélange d'eau et d'un ou plusieurs solvants cosmétiquement acceptables. De préférence, le liquide comprend au moins de l'eau, et encore plus préférentiellement il est de l'eau.

Un dispositif particulièrement utile pour la mise en oeuvre du procédé de la présente invention est une machine à café du type « expresso ». De telles machines sont bien connues de la technique. Par exemple, ces machines sont décrites dans les brevets AT 168405, US 2688911, DE 32433870 et IT 1265636.

Selon un mode de réalisation particulier de l'invention, l'étape de percolation est mise en oeuvre avec un fluide à une température supérieure à 30°C, de préférence comprise entre 30°C et 150°C, sous une pression comprise entre 3 et 30 bars, ou d'au moins 4 bars, de préférence supérieure à 10 bars et tout particulièrement comprise entre 10 et 30 bars.

La ou les vitamines, sous forme solide ou pâteuse, peuvent être utilisées directement, dans le dispositif générant le fluide sous pression, dans un récipient destiné à cet usage. Elles peuvent également être conditionnées dans un dispositif de conditionnement particulier du type monodose comprenant un logement fermé délimité par au moins une paroi au moins en partie perméable à un fluide sous pression d'au moins 3 bars. De tels dispositifs sont, par exemple, décrits dans les demandes de brevets WO00/56629, EP512470, US5897899 ou WO99/03753. Ces dispositifs de conditionnement sont en général étanches à l'air, l'humidité et/ou la lumière.

Selon un mode de réalisation particulier, le logement est délimité par deux feuilles scellées. Selon un autre mode de réalisation, le logement est délimité par une barquette fermée par un couvercle.

Ces dispositifs peuvent être fabriqués à partir de matériaux tissés ou non tissés, en matière plastique ou végétale, par exemple, en cellulose, en métal tel que l'aluminium ou en composite. De tels dispositifs sont par exemple décrits dans les demandes de brevets WO00/56629, EP512470, US5897899 ou WO99/03753.

Les vitamines utilisées selon l'invention sont de préférence choisies parmi la vitamine A, la vitamine B3, la vitamine B5, la vitamine B9, la vitamine C, la vitamine E, la vitamine F, la vitamine H, leurs dérivés, leurs précurseurs et leurs mélanges.

### Vitamine C

L'acide ascorbique ou vitamine C stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Ces deux dernières propriétés en font un excellent candidat comme actif cosmétique ou dermatologique pour lutter et/ou prévenir le vieillissement de la peau. La vitamine C permet aussi de protéger les cheveux contre les phénomènes oxydatifs notamment d'origine radicalaire.

La vitamine C correspond à l'acide ascorbique qui est généralement sous forme L, car il est habituellement extrait de produits naturels. Les dérivés de l'acide ascorbique sont, plus particulièrement, ses sels, tels que notamment l'ascorbate de sodium, l'ascorbylphosphate de magnésium ou de sodium ; ses esters, tels que notamment ses esters comme l'acétate d'ascorbyle, le nicotinate de tocophérol, le palmitate d'ascorbyle et le propionate d'ascorbyle, ou ses sucres, tels que notamment l'acide ascorbique glycosylé, le succinate de tocophérol, l'ascorbyl phosphate de magnésium, les sels tels que l'ascorbate de sodium, et leurs mélanges.

Les vitamines B3 et B5 peuvent réagir pour traiter ou prévenir le vieillissement de la peau, et également pour enlever l'aspect huileux de la peau, rendre régulières la texture de la peau et et/ou la taille des pores de la peau, et/ou traiter les peaux grasses. Elles permettent aussi d'obtenir des cheveux brillants à l'aspect sain et de stimuler leur croissance ou de ralentir leur chute.

### Vitamine B3

La vitamine B3, encore appelée vitamine PP est un composé de formule 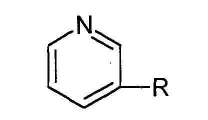 dans laquelle R peut être -CONH2 (niacinamide), -COOH (acide nicotinique ou niacine), ou CH2OH (alcool nicotinyle), -CO-NH-CH2-COOH (acide nicotinurique) ou -CO-NH-OH (acide niconityl hydroxamique).

Comme dérivés de la vitamine B3, on peut citer par exemple les esters de l'acide nicotinique tels que le nicotinate de tocophérol, les amides dérivés de la niacinamide par substitution des groupes hydrogène de - CONH2, les produits de réaction avec les acides carboxyliques et les acides aminés, les esters d'alcool nicotinyle et d'acides carboxyliques tels que l'acide acétique, l'acide salicylique, l'acide glycolique, l'acide palmitique. On peut citer aussi les dérivés suivants : 2-chloronicotinamide, 6-méthylnicotinamide, 6-aminonicotinamide, N-méthyl-nicotinamide, N,N-diméthylnicotinamide, N-(hydroxyméthyl)-nicotinamide, imide d'acide quinolinique, nicotinanilide, N-benzylnicotinamide, N-éthylnicotinamide, nifénazone, nicotinaldéhyde, acide isonicotinique, acide méthylisonicotinique, thionicotinamide, nialamide, acide 2-mercaptonicotinique, nicomol et niaprazine, le nicotinate de méthyle, le niconitate de sodium.

Comme autres dérivés de la vitamine B3, on peut citer également ses sels inorganiques tels que les chlorures, bromures, iodures, carbonates, et ses sels organiques tels que les sels obtenus par réaction avec des acides carboxyliques tels que acétate, salicylate, glycolate, lactate, malate, citrate, mandélate, tartrate, etc).

### Vitamine B5

Comme vitamine B5, on peut utiliser le panthénol ou alcool panthénylique ou 2,4-dihydroxy-N-(3-hydroxypropyl)-3,3diméthylbutanamide, sous ses différentes formes : D-panthénol, DL-panthénol, et ses dérivés et analogues, tels que le panthoténate de calcium, la panthétine, la pantothéine, le dipenthényl d'éthyl éther, l'acide pangamique, la pyridoxine, le pantoyl lactose, et les composés naturels en contenant tels que la gelée royale.

On peut également utiliser la vitamine B9, ou acide folique.

### Vitamine D

La vitamine D est une vitamine essentielle pour la prévention et le traitement des défauts de minéralisation du cartilage (rachitisme) et de l'os (ostéomalacie), et même de certaines formes d'ostéoporose chez le sujet âgé. Mais ces fonctions s'étendent bien au-delà de la régulation du métabolisme osseux et de l'homéostasie calcique. Parmi celles-ci, peuvent être citées ses actions sur la prolifération et sur la différenciation cellulaire et le contrôle des défenses immunitaires. En application topique, la vitamine D et ses analogues permettent notamment de traiter les désordres de la peau comme par exemple le psoriasis et les signes du vieillissement.

On peut citer comme vitamine D la 1α,25-dihydroxyvitamine D3 et ses analogues, ainsi que les analogues de vitamine D, tels que ceux décrits dans le document WO-A-00/26167, comme par exemple :
- 3-hydroxyméthyl-5-{2-[3-(5-hydroxy-5- ou 6-méthyl-hexyl)-phényl]-vinyl}-phenol,
- 3-[3-(5-hydroxy-1,5-diméthyl-hexyl)-phenoxyméthyl]-5-hydroxyméthyl-phenol,
- 6-[3-(3,4-bis-hydroxyméthyl-benzyloxy)-phényl]-2-méthyl-hepta-3,5-dien-2-ol,
- 6-[3-(3,4-bis-hydroxyméthyl-benzyloxy)-phényl]-2-méthyl-hexan-2-ol,
- 6-[3-(3,4-bis-hydroxyméthyl-phenoxyméthyl)-phényl]-2-méthyl-heptan-2-ol,
- 7-[3-(3,4-bis-hydroxyméthyl-phénoxyméthyl)-phényl]-3-éthyl-octan-3-ol,
- 5-{2-[4-(5-hydroxy-5-méthyl-hexyl)-phényl]-vinyl ou -éthyl}-benzène-1,3-diol,
- 5-{2-[3- ou 4-(6-hydroxy-6-méthyl-heptyl)-phényl]vinyl}-benzene-1,3-diol,
- 5-{2-[3- ou 4-(6-hydroxy-6-méthyl-heptyl)-phényl]-éthyl}-benzène-1,3-diol,
- 2-hydroxyméthyl-4-{2-[3- ou 4-(5-hydroxy-5-méthyl-hexyl)-phényl]-vinyl}-phenol,
- 2-hydroxyméthyl-4-{2-[3 ou 4-(6-hydroxy-6-méthyl-heptyl)-phényl]-vinyl}-phénol,
- 2-hydroxyméthyl-4-{2-[3- ou 4-(5-hydroxy-5-méthyl-heptyl)-phényl]-éthyl}-phénol,
- 2-hydroxyméthyl-4-{2-[3- ou 4-(6-hydroxy-6-méthyl-heptyl)-phényl]-éthyl}-phénol,
- 2-hydroxyméthyl-5-{2-[4-(5-hydroxy-5-méthyl-hexyl)-phényl]-vinyl}-phénol,
- 6-[3-(3,4-bis-hydroxyméthyl-benzyloxy)-phényl]-2-méthyl-heptan-2-ol,
- 4-[3-(5-hydroxy-1,5-diméthyl-hexyl)-phenoxyméthyl]-2-hydroxyméthyl-phenol,
- 6-{3- ou 4-[2-(3,4-bis-hydroxyméthyl-phényl)-vinyl]-phényl}-2-méthyl-hexan-2-ol,
- 7-{4-[2-(3,4-bis-hydroxyméthyl-phényl)-vinyl]-phényl}-2-méthyl-heptan-2-ol,
- 5-{2-[3-(6-hydroxy-6-méthyl-heptyl)-phényl]-1-méthyl-vinyl}-benzène-1,3-diol,
- 5-{2-[3-(5-hydroxy-5-méthyl-hexyl)-phényl]-vinyl}-benzène-1,3-diol,
- 5-[3-(6-hydroxy-6-méthyl-heptyl)-phénoxyméthyl]-benzène-1,3-diol,
- 5-{2-[3-(7-hydroxy-7-méthyl-oct-1-enyl)-phényl]-vinyl}-benzene-1,3-diol,
- 5-{2-[3-(7-hydroxy-7-méthyl-octyl)-phényl]-vinyl}-benzène-1,3-diol,
- 4-{2-[3-(6-hydroxy-6-méthyl-heptyl)-phényl]-vinyl}-benzène-1,2-diol,
- 3-{2-[3-(6-hydroxy-6-méthyl-heptyl)-phényl]-vinyl}-phénol,
- 6-{3-[2-(3,5-bis-hydroxyméthyl-phényl)-vinyl]-phényl}-2-méthyl-hexan-2-ol,
- 3-{2-[3-(7-hydroxy-7-méthyl-octyl)-phényl]-vinyl}-phénol,
- 7-{3-[2-(3,5-bis-hydroxyméthyl-phényl)-vinyl]-phényl}-2-méthyl-heptan-2-ol,
- 7-{3-[2-(3,4-bis-hydroxyméthyl-phényl)-vinyl]-phényl}-2-méthyl-heptan-2-ol,
- 7-{3-[2-(4-hydroxyméthyl-phényl)-vinyl]-phényl}-2-méthyl-heptan-2-ol,
- 4-{2-[3-(7-hydroxy-7-méthyl-oct-1-enyl)-phényl]-vinyl}-benzene-1,2-diol,
- 7-[3-(3,4-bis-hydroxyméthyl-phényléthynyl)-phényl]-2-méthyl-heptan-2-ol,
- 5-{2-[3-(6-hydroxy-6-méthyl-hept-1-enyl)-phényl]-vinyl}-benzene-1,3-diol,
- 5-{2-[3-(7-éthyl-7-hydroxy-non-1-enyl)-phényl]-vinyl}-benzène-1,3-diol,
- 5-{2-[3-(7-hydroxy-1-méthoxy-1,7-diméthyl-octyl)-phényl]-vinyl}-benzène-1,3-diol,
- 5-{2-[3-(6-hydroxy-1-méthoxy-1,6-diméthyl-heptyl)-phényl]-vinyl}-benzène-1,3-diol,
- 5-{2-[3-(5-hydroxy-pentyl)-phényl]-vinyl}-benzène-1,3-diol,
- 5-{2-[3-(5-hydroxy-6-méthyl-heptyl)-phényl]-vinyl}-benzène-1,3-diol,
- 5-{2-[3-(6-hydroxy-7-méthyl-octyl)-phényl]-vinyl}-benzène-1,3-diol,
- 5-{2-[3-(5-hydroxy-6-méthyl-hept-1-enyl)-phényl]-vinyl}-benzène-1,3-diol,
- 5-{2-[3-(6-hydroxy-7-méthyl-oct-1-enyl)-phényl]-vinyl}-benzène-1,3-diol,
- 5-{2-[3-(1,6-dihydroxy-1,6-diméthyl-heptyl)-phényl]-vinyl}-benzène-1,3-diol,
- 5-{2-[3-(6-hydroxy-1,6-diméthyl-hept-1-enyl)-phényl]-vinyl}-benzène-1,3-diol.

### Vitamine F

La vitamine F permet de lutter notamment contre la sécheresse de la peau. Elle permet aussi de renforcer le cheveu et de stimuler sa croissance ou de ralentir sa chute.

La vitamine F est un mélange d'acides gras essentiels, c'est-à-dire d'acides insaturés possédant au moins une double liaison, tels que l'acide linoléique ou acide 9,12-octadécadiènoïque et ses stéréoisomères, l'acide linolénique sous forme α (acide 9,12,15-octadécatriènoïque) ou γ (acide 6,9,12- octadéca-triènoïque) et leurs stéréoisomères, l'acide arachidonique ou acide 5,8,11,14-eicosatétraènoïque et ses stéréosiomères.

On peut utiliser dans la composition de la présente invention la vitamine F, ou les mélanges d'acides insaturés possédant au moins une double liaison et notamment les mélanges d'acide linoléique, d'acide linolénique et d'acide arachidonique, ou les composés en contenant et notamment les huiles d'origine végétale en contenant, telles que par exemple l'huile de jojoba. On peut aussi utiliser la vitamine F sous forme de dérivés et en particulier sous forme d'esters tels que les esters de sucres et de vitamine F.

On peut également utiliser la vitamine H, également appelée biotine. On peut l'utiliser pour le durcissement des ongles.

La ou les vitamines sont de préférence choisies parmi la vitamine A, la vitamine C, la vitamine B5, la vitamine E et la vitamine F.

La ou les vitamines peuvent être mises en oeuvre en mélange avec un ou plusieurs adjuvants solides ou pâteux, et de préférence pulvérulents. Une fois la percolation terminée, ces adjuvants restent dans le percolateur. Les adjuvants peuvent être choisis parmi les argiles, les sels, les agents tensioactifs anioniques, cationiques, non-ioniques, amphotères, zwittérioniques, les épaississants naturels ou de synthèse, les billes de verre, la silice, le nylon, les cires, les pigments, l'alumine, le dioxyde de titane, les zéolithes, le polyméthylméthacrylate (PMMA), le chitosane, la maltodextrine, la cyclodextrine, les mono ou disaccharides comme le glucose, le saccharose, le sorbitol, le fructose, l'oxyde de zinc, de zirconium, les particules de résine comme la silicone ou les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, l'amidon, éventuellement modifié, les grains de maïs, les gommes de polydiméthylsiloxanè, le polyacrylamide, l'hydroxyapatite poreux, la soie, le collagène, la sciure de bois, la poudre de fucus, les farines ou extraits de blé, de riz, de pois, de lupin, de soja, d'orge, la polyvinylpyrrolidone réticulée, l'alginate de calcium, le charbon actif, les particules de poly(chlorure de vinylidène/acrylonitrile), notamment celles commercialisées sous la dénomination générale d' « Expancel® » par la société AKZO NOBEL sous les références particulières « Expancel® WE » ou « DE » Expancels, et leurs mélanges.

Lorsqu'un ou plusieurs adjuvants sont présents, le ou les vitamines sont présentes de préférence en une quantité allant de 0,5 à 99% en poids, mieux encore de 1 à 80 % en poids, et encore plus préférentiellement de 2 à 60 % en poids par rapport au poids total vitamine(s) et adjuvants.

Les plantes ou extraits de plantes utilisés et au travers desquels passe le fluide sous pression peuvent être soumis avant la percolation à un traitement tel qu'une torréfaction, un cryobroyage, ou une lyophilisation.

La composition de traitement cosmétique des matières kératiniques obtenue selon le procédé de l'invention contient outre la ou les vitamines et le(s) composant(s) du fluide, à savoir l'eau et/ou le(s) solvant(s) cosmétiquement acceptable(s), éventuellement tout ou partie du ou des adjuvants présents dans le mélange solide ou pâteux.

L'invention concerne également une composition susceptible d'être obtenue par le procédé selon l'invention, la composition pouvant être exempte d'agents conservateurs.

A partir du procédé de préparation de l'invention, on obtient une composition de traitement cosmétique des matières kératiniques qui peut être appliquée directement sur les matières kératiniques, ou qui peut être mélangée à un milieu cosmétiquement acceptable, ou encore au moins un additif classiquement utilisé en cosmétique pourra y être ajouté par un opérateur. On peut également mélanger au moins deux compositions obtenues par le procédé de l'invention. La composition de traitement cosmétique des matières kératiniques résultant éventuellement de mélange(s) et/ou addition(s) indiqués ci-dessus, sera dénommée ci-après composition finale de traitement cosmétique ou composition finale.

Un mode de réalisation particulier de l'invention consiste à appliquer la composition obtenue par l'intermédiaire d'un dispositif ne nécessitant pas d'intervention humaine et équipé éventuellement d'un moyen de refroidissement.

Un autre mode de réalisation particulier de l'invention consiste à ingérer la composition de traitement cosmétique obtenue selon le procédé de l'invention lorsque aucun problème de toxicité n'est connu dans la technique.

La quantité de vitamines présentes dans la composition finale de traitement cosmétique obtenue par le procédé de la présente invention est en général comprise entre 0,001 et 50 % en poids environ du poids total de la composition finale de traitement cosmétique, de préférence entre 0,005 et 30 %, et encore plus préférentiellement entre 0,01 et 20%.

Lorsque la composition cosmétique obtenue par le procédé de la présente invention est mélangée à un milieu cosmétiquement acceptable, le milieu est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les àlcanols inférieurs en C1-C4, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40% en poids par rapport au poids total de la composition finale, et encore plus préférentiellement entre 5 et 30% en poids.

Au moins un additif classiquement utilisé en cosmétique peut être également ajouté dans les compositions de traitement cosmétique obtenues selon le procédé de la présente invention. A titre d'exemples de tels additifs, on peut citer des agents tensioactifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que, par exemple, des huiles de silicone, des agents filmogènes, des céramides, des agents conservateurs, des agents opacifiants, mais aussi des huiles, des cires, des gommes, des pigments colorés ou nacrés.

Les additifs ci-dessus sont en général présents en quantité comprise pour chacun d'eux entre 0,01 et 20% en poids par rapport au poids de la composition finale.

Bien entendu, l'homme de l'art veillera à choisir ce ou ces éventuels composés de manière telle que les propriétés avantageuses attachées intrinsèquement à la composition cosmétique conforme à l'invention ne soient pas, ou substantiellement pas, altérées par la ou les adjonctions envisagées.

Le pH de la composition finale est généralement compris entre 3 et 12, et de préférence entre 5 et 11. Il peut être ajusté à la valeur désirée au moyen d'agents acidifiants ou alcalinisants habituellement utilisés en cosmétique ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 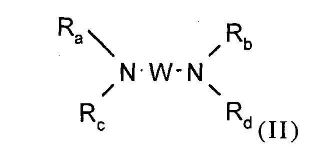 dans laquelle W est un reste propylène éventuellement substitué par un groupement hydroxyle ou un radical alkyle en C1-C4 ; Ra, Rb, Rc et Rd, identiques ou différents, représentent un atome d'hydrogène, un radical alkyle en C1-C4 ou hydroxyalkyle en C1-C4.

La composition finale de traitement cosmétique peut se présenter sous des formes diverses, telles que sous forme de liquides, de crèmes, de gels, de mascara ou sous toute autre forme appropriée pour réaliser un traitement des matières kératiniques, et notamment des fibres kératiniques, et de la peau. En particulier, la composition finale de traitement cosmétique peut être un rouge à lèvres contenant les vitamines A et E ou leurs dérivés, ayant un effet protecteur et anti-desséchant, et un rouge à lèvre contenant de la vitamine C pour un effet cicatrisant, contre les gerçures et les craquelures. La composition finale peut également être un fond de teint pour colorer la peau et/ou la protéger.

La présente invention concerne aussi un procédé de traitement cosmétique des matières kératiniques, comprenant la préparation d'une composition de traitement cosmétique selon le procédé tel que défini ci-dessus, et son application sur les matières kératiniques, par exemple par l'intermédiaire d'un opérateur ou par l'intermédiaire d'un dispositif ne nécessitant pas d'intervention humaine. La durée d'application peut varier entre 15 secondes et 1 heure.

Avant application, la composition de traitement cosmétique obtenue selon le procédé de l'invention peut être mélangée à un milieu cosmétiquement acceptable et/ou à un ou plusieurs additifs classiquement utilisés en cosmétique tels que décrits ci-dessus.

Un autre mode de réalisation consiste à préparer au moins deux compositions de traitement cosmétique selon le procédé de l'invention, à les mélanger, et à ajouter éventuellement un milieu cosmétiquement acceptable et/ou un ou plusieurs additifs classiquement utilisés en cosmétique tels que décrits ci-dessus, puis à appliquer la composition finale obtenue sur les matières kératiniques.

Les exemples ci-après sont destinés à illustrer la présente invention.

### Exemple 1 : préparation d'un après-shampooing

On mélange les ingrédients suivants :
- 2g de vitamine C en poudre commercialisée par la société Roche Vitamins
- 2g de bicarbonate d'ammonium
- 1 g de maltodextrine

On place ce mélange de 5g de poudre dans une machine expresso. On fait ensuite passer de la vapeur d'eau jusqu'à l'obtention d'une composition (A) présentant un volume final de 50 ml.

On peut ajouter ensuite à deux parties en poids de composition (A), une partie en poids d'une composition aqueuse (B) contenant 1 % en poids d'hydroxyéthylcellulose, afin de faciliter l'application.

Après application sur les cheveux, deux minutes de pose et un rinçage, on obtient des cheveux lisses et renforcés.

### Exemple 2 : préparation d'un gel anti-rides pour la peau

On prépare 5g de vitamine C en poudre commercialisée par la société Roche Vitamins.

On place cette poudre qui se trouve dans un récipient prévu pour recevoir un composé solide, dans une machine expresso. La poudre est ensuite traversée par la vapeur d'eau produite par la machine sous une pression de 3 bars. On introduit la composition obtenue, juste avant l'application sur la peau, dans un gel tamponné à pH 4, contenant :
Phase A
   - Carraghénane 1,5 g
   - Lubrajel (gélifiant) 28,5 g
   - Conservateur 0,2 g
   - Eau permutée qsp 95 g
   - Soude 0,5 g
Phase B
   - Eau permutée 4,8 g
   - Germall 115 0,3 g
   - Dequest 2046 0,1 g
   - Extrait d'aloès 0,5 g

Le gel est fabriqué de façon classique en préparant, séparément, par simple mélange des phases A et B respectivement à 70°C et 40°C, puis en ajoutant la phase B à la phase A sous agitation à 40°C et en laissant refroidir l'ensemble sous agitation lente jusqu'à la température ambiante. La composition obtenue permet un lissage du visage et une bonne hydratation de la peau.

## Revendications

1. Procédé de préparation d'une composition pour le traitement cosmétique des matières kératiniques, **caractérisé en ce qu'**il comprend une étape de percolation d'un fluide comprenant de la vapeur d'eau, sous une pression d'au moins 3 bars, au travers d'au moins une vitamine sous forme solide ou pâteuse.

2. Procédé selon la revendication 1, **caractérisé en ce que** la ou les vitamines sont choisies parmi la vitamine A, la vitamine B3, la vitamine B5, la vitamine B9, la vitamine C, la vitamine E, la vitamine F, la vitamine H, leurs dérivés, leurs précurseurs et leurs mélanges.

3. Procédé selon la revendication 2, **caractérisé en ce que** la ou les vitamines sont choisies parmi la vitamine A, la vitamine C, la vitamine B5, la vitamine E et la vitamine F.

4. Procédé selon l'une quelconque des revendications précédentes, **caractérisé en ce que** la vitamine sous forme solide ou pâteuse est mise en oeuvre en mélange avec au moins un adjuvant.

5. Procédé selon la revendication 4, **caractérisé en ce que** l'adjuvant est choisi parmi les argiles, les sels, les tensioactifs anioniques, non ioniques, cationiques ou zwittérioniques, les épaississants naturels ou de synthèse, l'amidon, éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono ou disaccharides, l'oxyde de zinc, de zirconium, les particules de résine comme la silicone ou les silicabades, le talc, l'acide polyaspartique, les borosilicates notamment de calcium, le polyéthylène, le coton, le polytétrafluoroéthylène (PTFE), la cellulose et ses dérivés, les composés superabsorbants, les carbonates de magnésium ou de calcium, les grains de maïs, les gommes de polydiméthylsiloxane, le polyacrylamide, l'hydroxyapatite poreux, la soie, le collagène, la sciure de bois, la poudre de fucus, les farines ou extraits de blé, de riz, de pois, de lupin, de soja, d'orge, la polyvinylpyrrolidone réticulée, l'alginate de calcium, le charbon actif, et les particules de poly(chlorure de vinylidène/acrylonitrile).

6. Procédé selon la revendication 4 ou 5, **caractérisé en ce que** la ou les vitamine sont présentes en une quantité allant de 0,5 à 99% en poids, de préférence de 1 à 80 %, et encore plus préférentiellement de 2 à 60% en poids par rapport au poids total vitamine(s) et adjuvant.

7. Procédé selon l'une quelconque des revendications précédentes, **caractérisé en ce que** l'étape de percolation est mise en oeuvre avec un fluide sous pression comprise entre 3 et 30 bars, de préférence comprise entre 10 et 30 bars.

8. Procédé selon l'une quelconque des revendications précédentes, **caractérisé en ce que** l'étape de percolation est mise en oeuvre avec un fluide sous pression d'au moins 10 bars.

9. Procédé selon l'une quelconque des revendications précédentes, **caractérisé en ce que** le fluide est de la vapeur d'eau, éventuellement accompagnée d'eau liquide.

10. Procédé selon l'une quelconque des revendications 1 à 9, **caractérisé en ce que** le fluide comprend un ou plusieurs solvants organiques liquides et/ou gazeux cosmétiquement acceptables.

11. Composition de traitement cosmétique susceptible d'être obtenue par la procédé selon l'une quelconque des revendications précédentes.

12. Composition selon la revendication 11, **caractérisée en ce qu'**elle est exempte de conservateur.

13. Procédé de traitement cosmétique des matières kératiniques, **caractérisé en ce qu'**on prépare une composition cosmétique selon le procédé défini selon l'une quelconque des revendications 1 à 10 et qu'on applique cette composition sur la matière kératinique.

14. Procédé de traitement cosmétique des matières kératiniques selon la revendication 13, **caractérisé en ce qu'**on applique la composition sur la matière kératinique par l'intermédiaire d'un dispositif ne nécessitant pas d'intervention humaine.

15. Procédé de traitement cosmétique des matières kératiniques selon la revendication 13, **caractérisé en ce que**, avant l'application, la composition de traitement cosmétique préparée selon le procédé selon l'une quelconque des revendications 1 à 10 est mélangée à un milieu cosmétiquement acceptable et/ou à un ou plusieurs additifs utilisés en cosmétique.

16. Procédé de traitement cosmétique des matières kératiniques, **caractérisé en ce que** l'on prépare au moins deux compositions cosmétiques selon l'une quelconque des revendications 1 à 10, on les mélange et on applique le mélange sur la matière kératinique.

17. Utilisation d'une composition obtenue par le procédé selon l'une quelconque des revendications 1 à 10 pour le traitement cosmétique des matières kératiniques.

18. Dispositif de conditionnement d'une composition cosmétique selon la revendication 11 ou 12, comprenant un logement fermé délimité par au moins une paroi au moins en partie perméable à un fluide sous pression d'au moins 3 bars, la composition contenant au moins une vitamine sous forme solide ou pâteuse.

19. Dispositif selon la revendication 18, **caractérisé en ce que** le logement est délimité par deux feuilles scellées.

20. Dispositif selon la revendication 18, **caractérisé en ce que** le logement est délimité par une barquette fermée par un couvercle.
